# EUROPEAN PATENT APPLICATION

(11) **EP 4 556 035 A1**
(43) Date of publication of application: **21.05.2025**
(21) Application number: 24151386.0
(22) Date of filing: 05.02.2024
(51) Int. Cl.: A61M 5/32, A61M 5/31

(54) **INJECTION DEVICE WITH A RESERVOIR HOLDER SENSOR**

(71) Applicant: Ypsomed AG, 3401 Burgdorf (CH)
(72) Inventor: Hostettler, Patrick, 3415 Hasle (CH); Etter, Leoni, 3011 Bern (CH)
(74) Representative: Kleiner, Stefan

(57) **Abstract**

The invention relates to an injection device (1) for dispensing a liquid drug from a reservoir (15) through a needle (18). The injection device (1) comprises a housing (87), a reservoir holder (30) releasably attachable to the housing (87), a movable needle cover (20), a capacitive sensor comprising a sensor area (93) and providing a capacitive signal and a controller connected to the sensor. The capacitive signal is indicative of a position of the reservoir holder (30) or of the needle cover (20) relative to the sensor area (93) and the controller is configured to determine a position of the reservoir holder (30) or the needle cover (20) relative to the housing (87) based on the capacitive signal.

## Description

### FIELD OF THE INVENTION

The present invention relates to an injection device for injecting, delivering, administering, infusing or dispensing substances and/or liquids such as insulin, hormone preparations or vaccines. The injection device includes a housing, a reservoir holder releasably attachable to the housing and a capacitive sensor.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a prefilled syringe as well as a drive mechanism in order to automatically move the plunger of the syringe upon actuation of the autoinjector. The drive mechanism typically comprises a source of drive, such as an electric motor or a mechanical spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector with the exception of the time when the needle is used for injection of a medicament. Thus, either the autoinjector moves the needle out of the housing for the injection and back into the housing after injection or the housing provides a needle guard which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the autoinjector.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the prefilled syringe with the needle and a needle cover sleeve. The user can thus discard the syringe unit when the syringe is empty or after use and can load the drive unit with a new syringe unit for a new upcoming injection.

Known approaches focus on monitoring the injection state or the presence or position of a syringe unit. EP 41121074 A1, for example, discloses a reusable autoinjector comprising a reusable drive unit and a disposable syringe unit releasably attachable to the drive unit. If a syringe unit is inserted into an opening in the drive unit to couple the syringe unit with the drive unit a position sensor detects the syringe unit and sends a corresponding signal to an electronic module inside the drive unit.

WO21170690 A1 discloses an injection device including a capacitive piston sensor. The sensor has a plurality of electrodes on opposite sides of a syringe barrel, so that the electrical properties of the barrel between these electrodes depend on a position of the syringe piston inside the barrel. In this way, the capacity value of the capacitive sensor that is formed with the electrodes corresponds to the fill level in the barrel or a position of the piston.

US 2013/0123685 A1 discloses an injection pen comprising capacitively based linear sensors. An electrode pattern is coupled to an annular track of a dose sleeve. A second electrode pattern is mounted on a lock item which follows an axial displacement of an injection button. The twoelectrode pattern cooperate with an electrode on a main electronic circuity so as to provide a sensor for monitoring dose setting and dose injections.

US2009096467 A1 discloses an injection pen comprising an assembly for determining the absolute position of rotatably mounted dose indicator sleeve relative to a pen housing. The indicator sleeve includes a scale of electrode elements which are capacitively coupled to receiver electrodes of an electronic unit in the injection pen.

### DESCRIPTION OF THE INVENTION

It is an objective of the invention to provide a reliable and adaptive position determination or presence detection of a reservoir holder or of a needle cover relative to a housing of an injection device.

This objective is achieved by an injection device or a method according to the independent claims. Preferred embodiments are evident from the dependent claims.

According to the invention an injection device for dispensing a liquid drug from a reservoir through a needle is provided. The injection device comprises
- a housing defining a longitudinal axis;
- a reservoir holder releasably attachable to a distal portion of the housing and adapted to hold the reservoir non-movable in a fixed position relative to the housing in an attached state;
- a needle cover movable relative to the reservoir holder (and relative to the housing) between a covering position in which the injection needle is covered and a retracted position in which the injection needle exposes;
- a capacitive sensor comprising a sensor area or sensor surface for sensing the reservoir holder or the needle cover, wherein the sensor provides a capacitive sensor signal;
- a controller electrically connected to the sensor.

The capacitive sensor is arranged such that the capacitive sensor signal of the capacitive sensor is dependent on a position (rotational or axial) of the reservoir holder or/and of the needle cover relative to the sensor area as the reservoir holder or/and the needle cover directly or indirectly (via intermediate member) interacts with the sensor area. The controller is configured to determine a position of the reservoir holder or/and the needle cover relative to the housing based on the capacitive sensor signal.

In contrast to known sensors for detection of a presence or position of a reservoir holder, reservoir assembly or needle cover the capacitive sensor according to the invention enables a reliable and in particular an adaptive position detection. As no mechanical switch (e. g. contact switch) has to be used the detection is robust and reliable against environmental influences and in particular against shocks if the device drops to the floor. Namely, the capacitive sensor according to the invention does not comprise any small or fragile moveable sensor element or switch element that can break, block or stick.

Furthermore, in contrast to a known mechanical switch the position detection according to the invention can be easily adjusted and is fully customizable regarding a trigger point along the longitudinal axis or in radial direction. That means due to the sensor area the position of the reservoir holder or needle cover can be detected in a predefined detection area and the controller can issue a user notification when the reservoir holder or the needle cover is located in a specific position within the detection area.

In contrast to fixedly installed switches the sensor arrangement according to the invention enables a sensor trigger level to be adjusted to the size or shape of the reservoir holder or of the needle cover. The sensor signal provided by the capacitive sensor can be used to determine an absolute position within the sensor area. That is useful as the housing may be connected to reservoir holders of different type or size and hence the final attachment position of the reservoir holder relative to the housing may be differ from one type of holder to another type of reservoir holder.

The above advantages are based on the fact that the reservoir holder or the needle cover (or an intermediate member coupled to the reservoir holder or needle cover) interacts with an electronic field provided by the sensor area (or of an electrode of the sensor area) and a detected change in capacitive coupling is indicative of (or represents) an absolute position or a presence of reservoir holder or of the needle cover.

The injection device may be either a manually actuated injection device or an automatic injection device. Automatic devices or autoinjectors typically include automatic drive means such as an elastic element (for example a pre-tensioned spring) or an electric motor to drive a dispensing member to dispense the liquid drug form the reservoir. Manual injection devices include a manual drive such as a dispensing button that has to be pressed by user force to move a dispensing member in dispensing direction.

The housing may accommodate a dose and dispensing mechanism including a manual or automatic drive. Preferably, the housing does not include any reservoir or drug container and has to be connected to the reservoir holder with a reservoir with the drug prior injection.

The reservoir holder is adapted to hold the reservoir, e.g., a syringe, cartridge, ampoule, with the liquid drug. The reservoir holder may include further components such as, for example, the needle cover, a support or a cap. The reservoir holder is preferably releasably attachable to the housing with its proximal portion. In particular a proximal reservoir holder portion can be inserted into an opening of the housing to couple the reservoir holder with the housing. This enables a replacement of an empty or used reservoir with a new reservoir to prepare the injection device for a new injection.

The capacitive sensor includes the sensor area and is adapted to measure the capacitance between two or more conductors (electrodes). The sensor area may include at least one active electrode providing an electric field. The controller is adapted to measure the strength or/and change of the capacitive coupling between the electrodes. Accordingly, the controller can determine the presence or a movement of the reservoir holder or of the needle cover when the reservoir holder, the needle cover or an intermediate member interacts with the electric filed and thus changes the capacitive coupling. In this way, a movement along the longitudinal axis, along a circumferential direction perpendicular to the longitudinal axis or a movement having radial as well as axial movement components, e.g., a helical movement may be sensed.

The capacitive sensor is preferably a contactless sensor and thus any mechanical wear between moving components can be avoided.

The controller is preferably arranged inside the housing, for example, in an electronic module. The controller may be a microcontroller or FPGA or any other signal processing unit.

Even though the function of the capacitive sensor according to the invention is described herein with an "or" between the reservoir holder and the needle cover it should be understood that the capacitive sensor is adapted to sense both the position or presence of the reservoir holder and of the needle cover at the same time or subsequently. Hence, the sensor may sense the position or presence of the reservoir holder or of the needle cover or of both, the reservoir holder as well as the needle cover.

The position detection or movement detection of the reservoir holder or the needle cover can be linked to each other. However, in a preferred embodiment the position detection of the reservoir holder and/or the needle cover is independent. For example, the sensor may firstly detect an attached reservoir holder and may secondly detect when the needle cover is moved from the covering position into the retracted position or vice versa. That means a movement of the reservoir holder relative to the housing during attachment may cause a first detectable change in the capacitive coupling and a possible later needle cover movement may cause a second detectable change in the capacitive coupling.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In a preferred embodiment the injection device further comprises a sensor trigger member which can cooperate, engage or about the reservoir holder and/or the needle cover and which trigger member is movable relative to the sensor area, for example, movable above the sensor area. A position of the sensor trigger member is thus representative of a reservoir holder position or/and a needle cover position relative to the housing. The sensor trigger member may thus act as an extension or connecting member which can link the position of the reservoir holder and/or the needle cover and the sensor area. However, such a sensor trigger member is not mandatory. In an alternative embodiment the reservoir holder and/or the needle cover may directly interact with the sensor area without any sensor trigger member.

The sensor trigger member can act as an extension such that the sensor area does not have to be located in close proximity to the reservoir holder or to the needle cover. Furthermore, the sensor trigger member may have a compact design enabling a compact design of the sensor area. The overall dimensions of the injection device can thus be optimized.

The sensor trigger member may be, for example, a rod, an arm or sleeve. Moreover, the sensor trigger member may be releasably or non-releasably connected to the reservoir holder or/and to the needle cover. Preferably, the sensor trigger member is movable, preferably exclusively along the longitudinal axis, relative to the housing.

Furthermore, the sensor trigger member may comprise a rod-shaped portion or extension extending along the longitudinal axis and may further comprise a trigger cam connected to the rod-shaped portion and positioned above the sensor area to interact with the sensor. Such a form enables a spacesaving design.

In a preferred embodiment the trigger cam is made of an electrically conductive material and is adapted to influence the capacitive coupling when the cam is moved above the sensor area. The material of the trigger cam may be, for example, iron, copper, aluminum or an alloy. The trigger cam is preferably not an active electrode and thus not connected to an electric circuit. In a preferred embodiment the trigger cam is cylindrical-shaped and made of full material.

Alternatively, the trigger cam is made of a non-conductive material or is an electric insulator, e. g. a dielectric, for example, made of ceramic, plastic or glass. A dielectric or insulating trigger cam influence the electrical field of the sensor area and thus a cam position can be detected by the capacitive sensor. The trigger cam may be made of plastics.

The sensor trigger member preferably includes further a contact portion, in particular a contact plate, extending in a plane perpendicular to the longitudinal axis. The portion is adapted to contact or engage a proximal end or end portion of the reservoir holder or/and the needle cover. That means if the reservoir holder is attached or about to be attached to the housing the contact portion is moved along the longitudinal axis and thus the trigger cam is moved above the sensor area. In the same way, when the needle cover is moved between the covering position and the retracted position the trigger cam is moved above the sensor area. As the trigger cam influences the capacitive coupling the controller can detect a presence or movement of the reservoir holder or/and the needle cover via trigger member.

The contact portion may be a plate or disc-shaped element extending in a plane perpendicular to the longitudinal axis.

The sensor trigger member and thus the contact portion is preferably biased by a spring member. That ensures that the contact portion is in contact with the reservoir holder or/and with the needle cover. The spring member may be a mechanical spring such as a torsion spring or spiral spring. Alternatively, the spring member may be a compressible elastic material such as an elastomer element.

In a preferred embodiment the sensor area comprises at least two electrodes which are arranged and shaped such that the capacitive sensor signal is unique (and thus unambiguous) for every possible position of the reservoir holder or of the needle cover relative to the sensor area. As the electromagnetic field provided by the electrodes depends on their geometric shape the form of the at least first and second electrode are preferably irregular or nonrecurrent along the longitudinal axis. The electrodes may be electrically separated by an insulating layer or barrier between a first and a second electrode.

That means a sensor signal value is an unequivocal function of the position of the reservoir holder and/or of the needle cover. That enables the controller to reliably determine a position of the reservoir holder or/and the needle cover relative to the housing. For example, the controller can reliably determine if the reservoir holder is correctly or completely attached to the housing or if the cover sleeve is completely moved into its retracted position.

In a further preferred embodiment, the sensor area comprises at least three electrodes. Three or more electrodes enable an elongated sensor area along the longitudinal axis and thus allow to precisely sense a longer movement along the longitudinal direction.

Preferably, a first pair of two adjacent electrodes define a first geometric form and a second pair of two adjacent electrodes define a second geometric form which is different from the first geometric form.

The two electrodes forming a first pair may have a different shape or they may be identical. Moreover, the same electrode may be part of the first as well as the second pair. However, a first geometric shape formed by the first pair of electrodes is different than a second geometric shape formed by the second pair of electrodes. That means the electromagnetic field provided by the electrodes is nonrecurrent and thus the sensor signal is unique for every position along the longitudinal axis.

In a preferred embodiment the sensor area comprises a pattern of electrodes that is arrowhead-shaped or triangular-shaped along the longitudinal axis. The arrowhead-shaped pattern provides a nonrecurrent electromagnetic field along the longitudinal axis as mentioned above.

As an alternative to the arrowhead-shaped pattern the electrode may have, for example, a rectangularshaped, circular-shaped or it may have a free irregular shape or organic geometry.

In the embodiment where the sensor area comprises two or more electrodes adjacent along the longitudinal axis a gap is present between two electrodes. A gap between a first and a second electrode is preferably arrowhead-shaped along the longitudinal axis.

A length of the sensor area along the longitudinal axis is preferably equal or longer than a maximal travel path of the needle cover and preferably of the reservoir holder relative to the housing. That means any possible position along the longitudinal axis of the needle cover and/or of the reservoir holder is detectable by the sensor according to the invention.

Preferably, the controller is configured to start an automatic drive to dispense the liquid drug from the reservoir when the sensor detects that the needle cover is in its retracted position. That means if the user places the injection device onto the injection site and presses the device against the injection site the needle cover is moved from its covering position into its retracted position. Thereby the needle cover (or a sensor trigger member, if any) is moved above the sensor area and the controller can determine when the needle cover is in its the retracted position. Subsequently, the controller can start the dispensing by driving a dispensing member (such as a plunger rod) in dispensing direction. Such a release mechanism is called push-on-skin release.

In a preferred embodiment the injection device comprises a preassembled disposable syringe unit and a preassembled reusable drive unit releasably attachable to the syringe unit. The syringe unit preferably comprises the needle cover and the reservoir holder and the drive unit comprises the housing, the sensor trigger member and an electromechanical drive for dispensing the liquid drug from the reservoir.

The syringe unit may be preassembled. That means the needle cover and the reservoir holder are connected to each other forming the syringe unit. The syringe unit can thus be stored and handled separately from the drive unit.

Preferably, the proximal end of the reservoir holder is in the same plane as the proximal end of the needle cover when it is in its covering position. Hence, during attachment of the syringe unit to the drive unit the reservoir holder (or a sensor trigger member, if any) is moved over the sensor area which can be sensed as described above. After attaching, the needle cover may be moved from its covering position into its retracted position and thereby the needle cover (or the sensor trigger member) is moved over the sensor area.

The needle cover is preferably movably mounted on the reservoir holder. The needle cover is preferably guided by the reservoir holder. When the syringe unit is attached to the drive unit the reservoir holder (or a sensor trigger member if any) may interact with the sensor area and thus the position can be detected as described above. As the needle cover is supported by the reservoir holder the same sensor area can be used to detect a needle cover position and/or a needle cover movement relative to the housing. That means the capacitive sensor provides not only a signal representing the reservoir holder position but additionally a signal indicative of a position or movement of the needle cover.

Sensor is not only adapted to sense the reservoir holder position (and hence the syringe unit position relative to the drive unit) but the sensor can also sense if the needle cover is in the covering position or in the retracted position.

The syringe unit preferably includes the reservoir in form of a prefilled syringe that has a needle or cannula permanently attached to a first end of a syringe barrel. The latter is sealed at the opposing end by a plunger. The injection device is preferably adapted to dispense the whole content of the syringe in a single injection stroke by the electromechanical drive.

Such types of injection device with a syringe unit and a drive unit are named as reusable autoinjector or semi-disposable autoinjector. In these semi-reusable injector concepts the syringe unit is typically not operational without a reusable drive unit of the injection device. To prepare the injection device for an injection the user has to attach or to couple the syringe unit to a drive unit. The syringe unit is usually attachable to the drive unit by means of a releasable lock such as a snap-fit connection, a thread or a bayonet fitting. After the injection or if the syringe is empty the user disconnects the syringe unit and can attach a new syringe unit to the drive unit.

In a preferred embodiment the needle cover is preferably the outermost part of the syringe unit. That means the syringe unit does not comprise any outer housing or shell but only the movable needle cover. This provides for a simple design.

The controller is preferably configured to detect an attachment position of the syringe unit based on the capacity information. Preferably, the injection device further comprises a movable locking member and the controller is configured to move the locking member by the electromechanics drive into a locking position to lock the attached syringe unit to the drive unit.

The locking member ensures that the device is operable and that the syringe unit is not unintentionally removed from the drive unit. The controller may be configured to lock the syringe unit automatically to the drive unit upon detection of the attachment.

The invention further relates to a drive unit of an injection device for releasable attachment to a reservoir holder of an injection device adapted to dispense a liquid drug from a reservoir through an injection needle. The drive unit comprises
- a housing defining a longitudinal axis;
- a connecting element for releasable attachment of a reservoir holder to the housing;
- a capacitive sensor comprising a sensor area;
- a controller connected to the sensor,
- a sensor trigger member movable relative to the housing and representing a reservoir holder position or a needle cover position relative to the housing.

The capacitive sensor is arranged such that a capacitive sensor signal of the capacitive sensor is dependent on a position of the sensor trigger member relative to the sensor area and wherein the controller is configured to determine a position of the sensor trigger member relative to the housing based on the capacitive sensor signal.

Furthermore, the invention relates to a method for determining a position of a reservoir holder of a preassembled syringe unit relative to an injection device housing of a preassembled reusable drive unit attachable to the syringe unit, the method comprising the steps of
a) Inserting a reservoir holder into the injection device housing;
b) Moving, by the reservoir holder, a sensor trigger member relative to a sensor area of a capacitive sensor along a longitudinal axis of the device;
c) Sensing, by the capacitive sensor, a change in the capacitance caused by the sensor trigger member movement and
d) Determining, by a controller, a position of the reservoir holder relative to the housing based on the change in capacitance.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of a reusable autoinjector comprising a syringe unit and a drive unit;
- Fig.2: depicts a perspective view of the syringe unit;
- Fig.3: depicts an exploded view of the syringe unit;
- Fig.4: depicts a sectional view of the drive unit;
- Fig. 5: depicts a sectional view of the drive unit with an attached syringe unit;
- Fig. 6: depicts a sectional view of the autoinjector when the needle cover is in a retracted position and
- Fig. 7a-7d: depict schematical views of electrodes of a capacitive sensor according to the invention.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

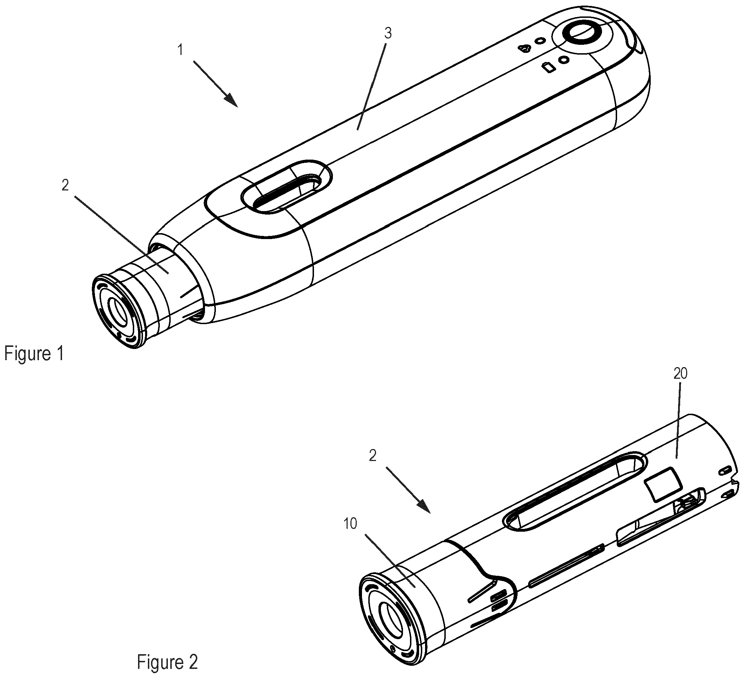

In the present description the term "distal" refers to the side where the injection needle is located. This is on the left-hand side in the figures. The term "proximal" refers to the opposite side or rear end of the device and is on the right-hand side in the figures 1 to 6.

Figure 1 shows a perspective view of an injection device according to the invention in form of a semi-reusable autoinjector 1. The autoinjector 1 comprises a preassembled disposable syringe unit 2 including a reservoir holder and a preassembled reusable drive unit 3 comprising a drive for dispensing a liquid drug from the reservoir. As shown in figure 1 the drive unit 3 has an elongated housing defining a longitudinal axis and includes an opening adapted to accommodate a proximal portion of the syringe unit 2 to releasably attach the syringe unit 2 to the drive unit 3.

In the following the relevant features of the syringe unit 2 and the drive unit 3 for the capacitive sensor according to the invention will be described. Subsequently, the function of the semi-reusable autoinjector 1 with respect to the sensor will be described.

Figure 2 depicts a perspective view of the syringe unit 2 alone without the drive unit. The syringe unit 2 has an elliptical form in the cross section and extends along the longitudinal axis. On a distal end a cap 10 is mounted in an initial state or shipping state. The proximal end includes a holding structure to releasably attach the syringe unit 2 to the drive unit 3.

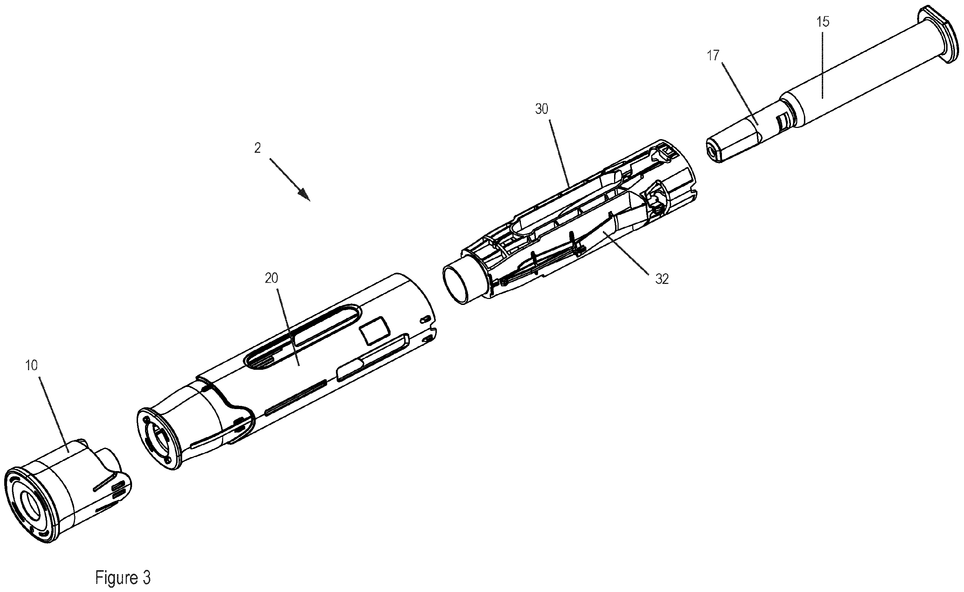

Figure 3 depicts an exploded view of the syringe unit 2. As shown in figure 3 the syringe unit 2 includes a syringe holder 30 (reservoir holder), a cover sleeve 20 coaxially arranged around the syringe holder 30 and movable relative thereto along the longitudinal axis, a label wrapped around the cover sleeve 20, the cap 10 with a RNS remover and a prefilled syringe 15 comprising a liquid drug and a rigid needle shield 17 (RNS) initially mounted on the injection needle. The prefilled syringe 15 with a barrel made of glass is rotationally and axially fixed inside the sleeve-shaped syringe holder 30.

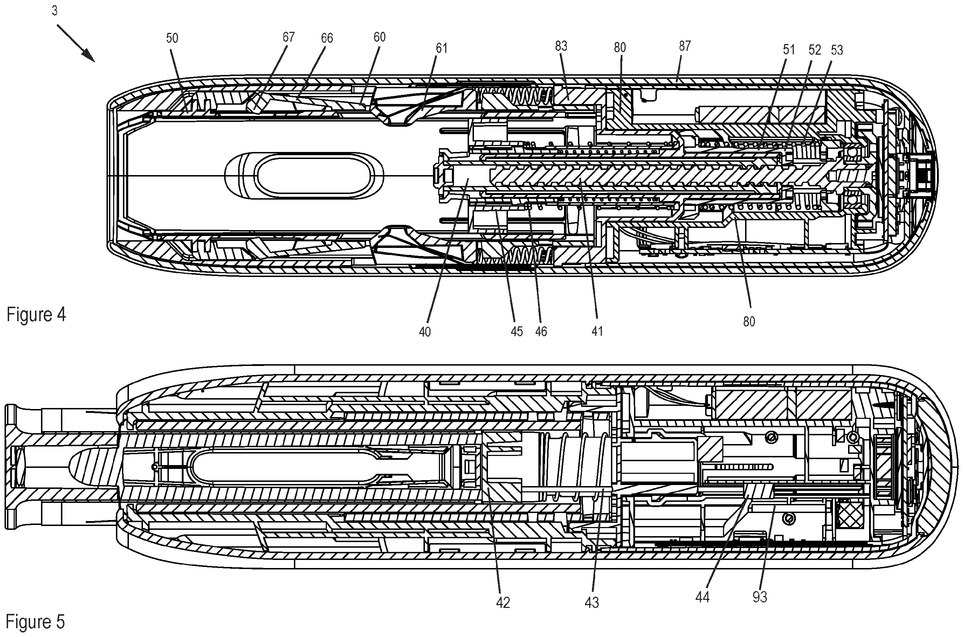

Figure 4 depicts a sectional view of the drive unit 3 without the syringe unit. The cut of the sectional view runs along the longitudinal axis. The drive unit 3 comprises a support structure 80 including an outer housing 87 and inside the housing a mechanics sleeve 83 is fixedly connected to the support structure 80. Furthermore, the drive unit 3 includes the opening on its distal side adapted to accommodate a proximal portion of the syringe unit 2.

The support structure 80 holds a gripper sleeve 60 which is immovably arranged with respect to the housing 87. Coaxially and inside the gripper sleeve 60 a trigger sleeve 50 is arranged which is movable relative to the supporting structure 80 along the longitudinal axis. Inside the trigger sleeve 50 a movable sensor trigger member in form of a cover sleeve connector 45 is located and biased in distal direction by a cover sleeve spring 46. The spring abuts on its distal end the cover sleeve connector 45 and on its proximal end a radial wall of the mechanics sleeve 83.

Figure 5 depicts a sectional view of the autoinjector with a syringe unit 2 attached to the drive unit 3. As shown in figure 5 the cover sleeve connector 45 comprises a contact plate 42 on a distal end extending in a plane perpendicular to the longitudinal axis. Furthermore, the cover sleeve connector 45 includes a distal rod-shaped portion 43 extending along the longitudinal axis. In a proximal portion of the rod a sensor trigger cam (sensor trigger) 44 is arranged and connected to the rod 43. The cam 44 is rectangular shaped and adapted to be moved above a sensor area or sensor surface 93 of a capacitive sensor. The contact plate 42, the rod portion 43 and the cam 44 are fixedly connected to each other (or made of a monolithic piece) and movable together along the longitudinal axis. The cam 44 is made of full material and made of metal. In an alternative embodiment the cam 44 may be made of plastics.

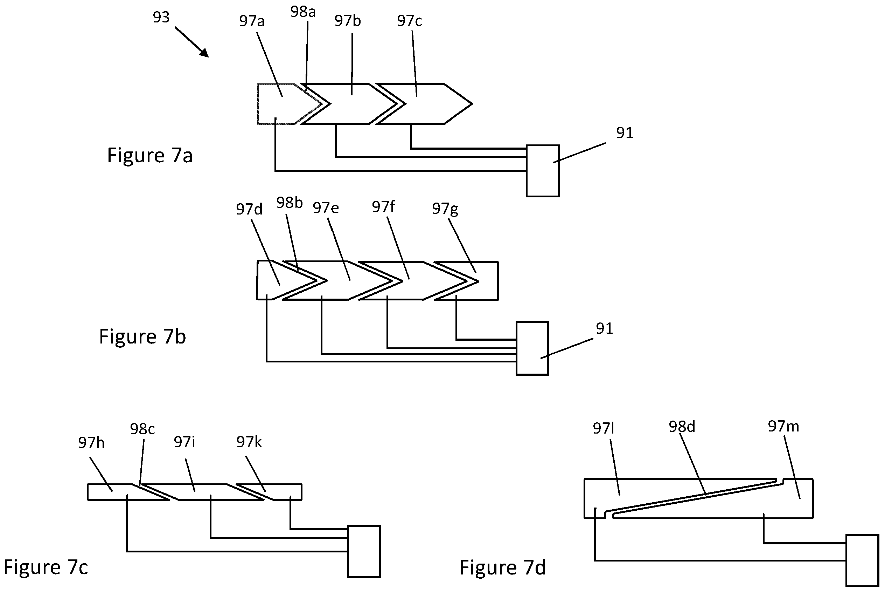

The capacitive sensor is arranged inside a proximal part of the drive unit housing 87. The sensor comprises sensor electronics 91 (including a microcontroller) and electrode plates 97a - 97c forming the sensor area 93 in the first embodiment as shown in figure 7a. The electrode plates 97a - 97c are directly positioned onto a PCB of the drive unit 3.

The sensor area 93 extends along the longitudinal axis. In the first embodiment the sensor area 93 is formed by three electrode plates 97a, 97b, 97c made of copper and arranged adjacent to each other along the longitudinal axis. The axial length of the sensor area corresponds to the maximum axial travel path of the cover sleeve of the syringe unit as described in detail below.

Each electrode plate is connected to the sensor electronics 91 which supplies each electrode to generate an electric field. The sensor electronics 91 are configured to sense a change in the capacitive coupling. Namely, when the sensor trigger cam 44 is moved above the sensor area 93 the capacitive coupling is influenced and the electronics are able to measure a change in a displacement current accordingly. A controller in the sensor electronics 91 can thus determine a position of the trigger cam 44 relative to the sensor area 93 and hence relative to the housing 87.

Figures 7a to 7d depict different embodiments of the sensor electrode plates. In the first embodiment shown in figure 7a the sensor area 93 includes three electrode plates 97a, 97b and 97c which are separated from each other by a line of an electric isolation 98a. As shown in figure 7a the isolation line 98a runs in an arrowhead-shape from one side to the other side. That means the first electrode plate 97a has an arrowhead shape or a triangular shape and the second and third electrode plate 97b, 97c have a corresponding arrowhead geometry. Thus, each electrode 97a - 97c has a nonrecurrent pattern along the longitudinal axis. The geometry of the electrodes shown in figure 7a are preferred. However, alternative shapes are possible as shown in figures 7b to 7d.

Figure 7b depicts a second embodiment with a sensor area including total four electrode plates 97d - 97g that are separated by arrowhead-shaped isolation lines 98b.

In a third embodiment shown in figure 7c three electrodes 97h, 97i, 97k are separated by a sloped isolation line 98c such that the middle electrode 97i has the shape of a parallelogram.

Finally, figure 7d depicts a fourth embodiment with two electrode plates 97l and 97m. The isolation line 98d is sloped such that the first electrode plate 97l has a triangular shape and the second electrode plate 97m has a corresponding counter triangular shape.

As the electric field directly depends on the geometry of the electrode plates the capacity of each of the above shown embodiments of figure 7a to 7d is thus different. As the form of the electrode plates varies along the longitudinal axis and the change of the capacitive coupling caused by the sensor trigger cam 44 can thus be assigned to a specific sensor trigger cam 44 position. That means, in turn, the controller is able to determine a position of the sensor trigger cam 44 relative to the sensor area 93 and hence relative to the drive unit housing 87.

In the following the function of the capacitive sensor is descripted.

In order to prepare the autoinjector for an injection the user attaches a syringe unit 2 to the drive unit 3. For that purpose, a proximal portion of the syringe unit 2 is inserted into the opening in drive unit 3. The syringe unit 2 is hold in the drive unit by clamping arms 61 see figure 4. However, the syringe unit 2 is not yet locked and can be pulled out by the user at this stage.

When the syringe unit 2 is inserted into the drive unit 3 the proximal end of the syringe unit abuts a distal surface of the contact plate 42 (figure 5) of the cover sleeve connector 45 and subsequently moves the cover sleeve connector 45 a short distance of several millimeters in distal direction.

That in turn causes the sensor trigger cam 44 on the connector rod to move above the sensor area 93. During that movement the sensor trigger cam 44 influences the capacitive coupling of the sensor electrode plates 97a - 97c. Based on the sensed change the controller determines the position of the cam 44 relative to sensor area 93.

As mentioned above the electrode plates generate an electrode pattern-specific electric field along the longitudinal axis. For each possible cam position the controller can determine an absolute position of the cam 44 and thus of the cover sleeve connector 45 (and thus of the needle cover sleeve 20 and the syringe holder 30) based on the capacitive sensor signal.

When the controller detects that a syringe unit 2 has been inserted and completely attached to the drive unit 3 based on a movement of the cover sleeve connector 45 the controller locks the syringe unit 2 to the drive unit 3. For this purpose, the controller rotates the motor to rotate a pinion and the gear transfers the rotation to a threaded rod 41 (figure 4) which in turn moves a non-rotating plunger rod 40 in proximal direction, preferably an axial travel of about 5 mm. As the trigger sleeve connector 51 is connected via its ledges 52 to a proximal shoulder of the plunger rod 40 the latter causes the trigger sleeve connector 51 to move and thus the trigger sleeve 50 is shifted in proximal direction too. Furthermore, the trigger sleeve spring 53 is compressed during the proximal movement.

The proximal movement of the trigger sleeve 50 causes actuation arms 66 to deflect radially inwards and actuation arms free end 67 is moved radially inwards when the trigger sleeve 50 is further moved in proximal direction. A radial inward directed contact surface of the free ends 67 of the deflected actuation arms 66 in turn abut release arms 32 thereby deflect the release arms 32 radially inwards too.

The radially inwardly deflected release arms 32 have the effect that they no longer engage a stop surface of the cover sleeve 20 and thus allow for the cover sleeve 20 to move out of a covering position and move inside the support structure 80 in proximal direction relative to the syringe holder 30.

At the same time the radially inwards deflected actuation arms 66 abut a distal stop surface (not shown) of the syringe holder 30 and thereby lock the syringe holder 30 relative to gripper sleeve 60 and hence to the support structure 80. Thus, the syringe unit 2 can no longer pulled out of the drive unit 3.

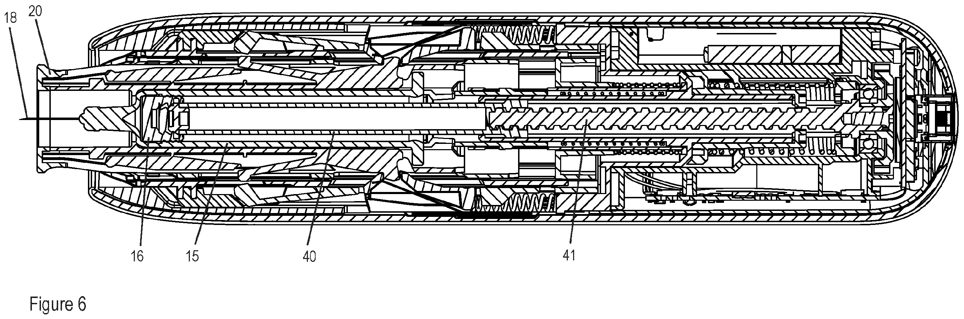

As a next step the user places the distal end of the cover sleeve 20 onto an injection site and pushes the autoinjector 1 towards the injection site. This causes the cover sleeve 20 to move proximally from the distal covering position into support structure 80 (push on skin) and compresses the cover sleeve spring 46 which biases the cover sleeve 20 in distal direction via cover sleeve connector 45. When the cover sleeve 20 is in its retracted position the injection needle 18 protrudes from the cover sleeve as shown in figure 6.

Upon push on skin the cover sleeve connector 45 is moved proximally and thus the sensor trigger cam 44 is further moved proximally. The further movement is detected by the capacitive sensor as the cam 44 moves over the sensor area 93 and influences the capacitive coupling described above.

That means based on the capacitive change the controller can determine when the cover sleeve 20 is in the retracted position and can start the injection.

Consequently, the controller drives the electric motor in dispensing direction and thus rotates the threaded rod 41 and thereby moves the plunger rod 40 in distal direction to dispense the liquid drug from the syringe.

After the injection the user removes the autoinjector from the injection site and due to the cover sleeve spring 46 the cover sleeve 20 is moved back into its covering position. As this causes the sensor trigger cam 44 to move again above the sensor area 93 in distal direction the cover sleeve return movement can be detected by the controller.

In summary, the cover sleeve connector 45 and thus the sensor trigger cam 44 are moved twice during the preparation of the autoinjector: a first time during attachment of the syringe unit 2 generating a signal that the syringe unit 2 has been completely attached and a second time when the user pushes the device onto the skin and the cover sleeve 20 moves the cam 44 further proximally. That means a single capacitive sensor can be used for both a syringe unit detection and for a cover sleeve position determination, respectively.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | autoinjector | 50 | trigger sleeve |
| 2 | syringe unit | 51 | trigger sleeve connector |
| 3 | drive unit | 52 | ledge |
| | | 53 | trigger sleeve spring |
| 10 | cap | | |
| 15 | prefilled syringe | | |
| 16 | piston | 60 | gripper sleeve |
| 17 | rigid needle shield RNS | 61 | clamping arms |
| 18 | injection needle | 66 | actuation arm |
| | | 67 | free end |
| 20 | cover sleeve | | |
| 30 | syringe holder | 80 | support structure |
| 32 | release arm | 83 | mechanic sleeve |
| | | 87 | housing |
| 40 | plunger rod | | |
| 41 | threaded rod | 91 | sensor electronics |
| 42 | contact plate | 93 | sensor area |
| 43 | rod-shaped portion | 97a-m | electrode plates |
| 44 | sensor trigger cam | 98a-d | isolation |
| 45 | cover sleeve connector | | |
| 46 | cover sleeve spring | | |

## Claims

1. An injection device (1) for dispensing a liquid drug from a reservoir (15) through a needle (18), the injection device (1) comprising
• a housing (87) defining a longitudinal axis;
• a reservoir holder (30) releasably attachable to the housing (87);
• a needle cover (20) movable along the longitudinal axis relative to the reservoir holder between a needle covering position and a retracted position;
• a capacitive sensor comprising a sensor area (93);
• a controller connected to the sensor,
**characterized in that** the capacitive sensor is arranged such that a capacitive sensor signal of the capacitive sensor is dependent on a position of the reservoir holder (30) or of the needle cover (20) relative to the sensor area (93) and wherein the controller is configured to determine a position of the reservoir holder (30) or the needle cover (20) relative to the housing (87) based on the capacitive sensor signal.

2. Injection device (1) according to claim 1, further comprising a sensor trigger member (45) that can cooperate with the reservoir holder (30) or the needle cover (20) and is movable relative to the sensor area (93) such that a sensor trigger member (45) position is representative of the reservoir holder position or needle cover position relative to the housing.

3. Injection device (1) according to claim 2, wherein the sensor trigger member (45) comprises a rod-shaped portion (43) extending along the longitudinal axis including a trigger cam (44) positioned above the sensor area.

4. Injection device (1) according to claim 3, wherein the trigger cam (44) is made of an electrically conductive material.

5. Injection device (1) according to claim 3, wherein the trigger cam (44) is made of plastics.

6. Injection device (1) according to any of claims 2 to 5, wherein the sensor trigger member (45) is biased in distal direction by a spring member (46).

7. Injection device (1) according to any of claims 1 to 6, wherein the sensor area (93) comprises at least two electrodes (97a-97m) which are arranged such that the capacitive sensor signal is unique for every possible position of the reservoir holder (30) or of the needle cover (20) relative to the sensor area.

8. Injection device (1) according to claim 7, wherein the sensor area (93) comprises at least three electrodes (97a-97m), wherein a first pair of two adjacent electrodes define a first geometric form and a second pair of two adjacent electrodes define a second geometric shape which is different from the first geometric shape.

9. Injection device (1) according to claim 7 or 8, wherein the electrode (97a-97g) is arrowhead-shaped in the longitudinal axis.

10. Injection device (1) according to claim 7 or 9, wherein an insulation (98a) between a first and a second electrode (97a, 97b) is arrowhead-shaped in the longitudinal axis.

11. Injection device (1) according to any of claims 1 or 10, wherein a length of the sensor area (93) along the longitudinal axis is equal or longer than a maximal travel path of the reservoir holder (30) or the needle cover (20).

12. Injection device (1) according to any of claims 1 to 11, comprising a preassembled disposable syringe unit (2) and a preassembled reusable drive unit (3) releasably attachable to the syringe unit, wherein the syringe unit (2) comprises the needle cover (20) and the reservoir holder (30) and wherein the drive unit (3) comprises the housing (87), the sensor trigger member (45) and an electromechanical drive for dispensing the liquid drug from the reservoir (15).

13. Injection device (1) according to claim 12, wherein the controller is configured to detect an attachment position of the syringe unit (2) on the drive unit (3) based on the capacitive sensor signal and wherein the controller is configured to move a locking member (50) by the drive into a locking position to lock the syringe unit (2) to the drive unit (3).

14. A drive unit (3) for an injection device for dispensing a liquid drug from a reservoir (15) through a needle (18), the drive unit (3) comprising
• a housing (87) defining a longitudinal axis;
• a connecting element (61) for releasably attaching a reservoir holder (30) to the housing (87);
• a capacitive sensor comprising a sensor area (93);
• a controller connected to the sensor,
• a sensor trigger member (45) movable relative to the housing (87) and representing a reservoir holder position or a needle cover position relative to the housing,
**characterized in that** the capacitive sensor is arranged such that a capacitive sensor signal of the capacitive sensor is dependent on a position of the sensor trigger member (45) relative to the sensor area (93) and wherein the controller is configured to determine a position of the sensor trigger member (45) relative to the housing (87) based on the capacitive sensor signal.

15. Method for determining a position of a reservoir holder (30) of a preassembled syringe unit (2) relative to an injection device housing (87) of a preassembled reusable drive unit (3) attachable to the syringe unit, the method comprising the steps of
a) Inserting a reservoir holder (30) into the injection device housing (87);
b) Moving, by the reservoir holder (30), a sensor trigger member (45) relative to a sensor area of a capacitive sensor along a longitudinal axis of the device;
c) Sensing, by the capacitive sensor, a change in the capacitance caused by the sensor trigger member movement and
d) Determining, by a controller, a position of the reservoir holder (30) relative to the housing based on the change in capacitance.
